(19) Europäisches Patentamt
European Patent Office
Office européen des brevets

(11) **EP 3 145 587 B1**

(12) **FASCICULE DE BREVET EUROPEEN**

(45) Date de publication et mention
de la délivrance du brevet:
**27.06.2018 Bulletin 2018/26**

(21) Numéro de dépôt: **15728771.5**

(22) Date de dépôt: **22.05.2015**

(51) Int Cl.:
**A61N 5/10** $^{(2006.01)}$

(86) Numéro de dépôt international:
**PCT/EP2015/061393**

(87) Numéro de publication internationale:
**WO 2015/177343 (26.11.2015 Gazette 2015/47)**

(54) **MÉTHODE D'ESTIMATION DE LA DOSE DÉLIVRÉE PAR UN SYSTÈME DE RADIOTHÉRAPIE EXTERNE**

VERFAHREN ZUR SCHÄTZUNG DER VERABREICHTEN DOSIS VON EINEM EXTERNEN STRAHLENTHERAPIESYSTEM

METHOD FOR ESTIMATING THE DOSE ADMINISTERED BY AN EXTERNAL RADIOTHERAPY SYSTEM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.05.2014 FR 1454631**

(43) Date de publication de la demande:
**29.03.2017 Bulletin 2017/13**

(73) Titulaire: **SAS D.R.E.A.M.**
**31300 Toulouse (FR)**

(72) Inventeur: **BOUTRY-DUTHIL, Christine**
**31320 Mervilla (FR)**

(74) Mandataire: **Brevalex**
**56, Boulevard de l'Embouchure**
**B.P. 27519**
**31075 Toulouse Cedex 2 (FR)**

(56) Documents cités:
**US-B1- 8 130 905     US-B1- 8 605 857**

• **Benjamin E Nelms ET AL: "Evaluation of a fast method of EPID-based dosimetry for intensity-modulated radiation therapy", Journal of applied clinical medical physics / American College of Medical Physics, vol. 11, no. 2 1 janvier 2010 (2010-01-01), page 3185, XP055162371, United States Extrait de l'Internet: URL:http://www.ncbi.nlm.nih.gov/pubmed/205 92703 [extrait le 2015-01-15]**
• **VAN ELMPT W ET AL: "A literature review of electronic portal imaging for radiotherapy dosimetry", RADIOTHERAPY AND ONCOLOGY, ELSEVIER, IRELAND, vol. 88, no. 3, 1 septembre 2008 (2008-09-01), pages 289-309, XP025495448, ISSN: 0167-8140, DOI: 10.1016/J.RADONC.2008.07.008 [extrait le 2008-08-14]**

**Description**

## DOMAINE TECHNIQUE

La présente invention concerne de manière générale le domaine de la dosimétrie en radiothérapie externe. Elle trouve particulièrement à s'appliquer dans les systèmes de radiothérapie équipés d'un dispositif électronique d'imagerie portale.

## ÉTAT DE LA TECHNIQUE ANTÉRIEURE

Les systèmes de radiothérapie externe sont bien connus de l'état de la technique. Ils permettent de délivrer une dose de particules d'énergie élevée (de l'ordre de quelques MeV) à des tumeurs cancéreuses tout en épargnant les tissus sains voisins. Les effets ionisants de ces particules, que ce soit par ionisation directe lorsque ces particules sont chargées ou par ionisation indirecte lorsqu'elles ne le sont pas, provoquent la mort des cellules irradiées, par lésion des acides nucléiques responsables de la division cellulaire et de la synthèse des protéines.

Les systèmes de radiothérapie externe font appel à un accélérateur linéaire d'électrons. Dans un tel système, les électrons sont libérés par un canon à électrons puis accélérés par un champ alternatif haute fréquence produit par un magnéton ou un klystron. L'interposition d'une cible en tungstène conduit à la production d'un faisceau de photons pouvant atteindre une vingtaine de MeV. Traditionnellement, par abus de langage, on mesure généralement l'énergie des photons en MV en radiothérapie.

Le traitement d'une tumeur par radiothérapie externe consiste à délivrer une dose d'énergie donnée dans une zone d'étendue spatiale donnée. Afin d'épargner les tissus sains voisins, la dose est délivrée par irradiation sous plusieurs angles d'exposition prédéterminés, une dose élémentaire étant délivrée pour chaque angle. En outre, pour chaque angle d'exposition, le faisceau émis par la source de photons est collimaté selon un profil particulier de manière à réduire autant que possible l'irradiation des tissus sains. Il est donc nécessaire de planifier précisément les angles d'exposition, les profils de faisceau et les doses à délivrer pour chacun de ces angles. En pratique, le traitement est simulé au moyen d'une station informatique de planification de traitement ou TPS (*Treatment Planning Station*) pour chaque patient. Cette station permet de déterminer à partir d'images anatomiques tridimensionnelles du patient acquises à l'aide d'un scanner radiologique (CT), les angles d'exposition ainsi que le profil de faisceau et la dose pour chaque angle.

Cette technique de radiothérapie, adaptée à la géométrie tridimensionnelle de la tumeur est encore appelée « radiothérapie conformationnelle ». D'autres avancées techniques ont récemment vu le jour, notamment celle apportées par la radiothérapie par modulation (spatiale et temporelle) d'intensité du faisceau ou IMRT (*Intensity Modulated Radio Therapy*) ou l'arcthérapie VMAT (*Volumetric Modulated Arc Therapy*).

Quelle que soit la technique de radiothérapie envisagée, il est particulièrement important de vérifier que les doses effectivement délivrées au patient sont bien conformes à celles prévues lors de la planification du traitement, pour s'assurer non seulement que la dose totale délivrée à la tumeur est bien celle souhaitée mais aussi que la consigne de gradient de dose entre tissus cancéreux et tissus sains a bien été respectée. Une méthode de vérification de dose délivrée à un patient dans un système de radiothérapie conformationnelle est décrite dans la demande de brevet EP-A-2 248 551.

De nombreux matériels et logiciels sont actuellement disponibles sur le marché pour effectuer un contrôle dosimétrique. Les systèmes de radiothérapie externe récents sont généralement équipés d'un imageur portal numérique ou EPID (*Electronic Portal Imaging Device*). Un imageur portal est défini comme un dispositif adapté à acquérir des images à partir d'un champ de traitement en radiothérapie. Les images ainsi obtenues, dites images portales, montrent la zone irradiée et permettent de vérifier le positionnement du patient dans le champ de traitement.

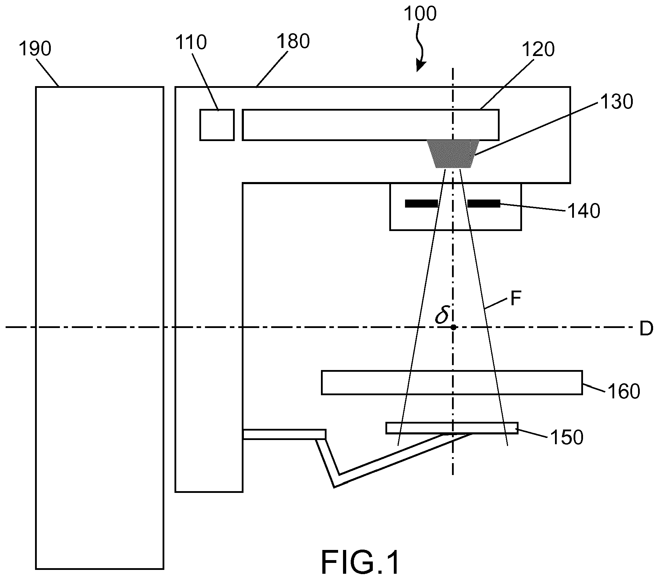

La Fig. 1 représente de manière schématique un système de radiothérapie externe équipé d'un imageur portal.

Le système de radiothérapie externe comprend un accélérateur linéaire, 120, alimenté par un canon d'électrons, 110 et se terminant par une tête d'accélérateur (non représentée). Le canon à électrons et l'accélérateur linéaire sont embarqués dans un bras mécanique 180, capable de tourner autour d'un axe D, solidaire d'un portique 190. Dans la tête de l'accélérateur sont notamment montés la cible 130, un système de collimation primaire, un cône égalisateur de faisceau et un système de collimation secondaire multi-lames, 140. La cible (en tungstène), bombardée par les électrons issus de l'accélérateur, constitue la source de photons X. L'imageur portal, 160, est également embarqué dans le bras mécanique, du côté opposé à la source de photons. Ainsi, lors d'une rotation du bras, l'imageur portal se trouve toujours placé dans un plan orthogonal à l'axe principal du faisceau. En cours de traitement, le patient est allongé sur une table, dite table de traitement, 160, située entre la source et l'imageur portal. Le point d'intersection, $\delta$, entre l'axe de rotation du bras mécanique, D, et l'axe principal du faisceau, $F$, est appelé isocentre du système.

La dernière génération d'imageur portal fait appel à un détecteur à silicium amorphe. Il existe différents types de détecteurs à silicium amorphe, ceux à détection directe et ceux à détection indirecte. Ceux à détection directe n'utilisent pas d'écran fluorescent mais directement un photoconducteur qui convertit les rayons X en charges électriques.

En revanche, ceux à détection indirecte utilisent un écran fluorescent pour convertir les photons incidents en photons visibles, ces photons visibles étant ensuite convertis en charges électriques formant le signal à mesurer.

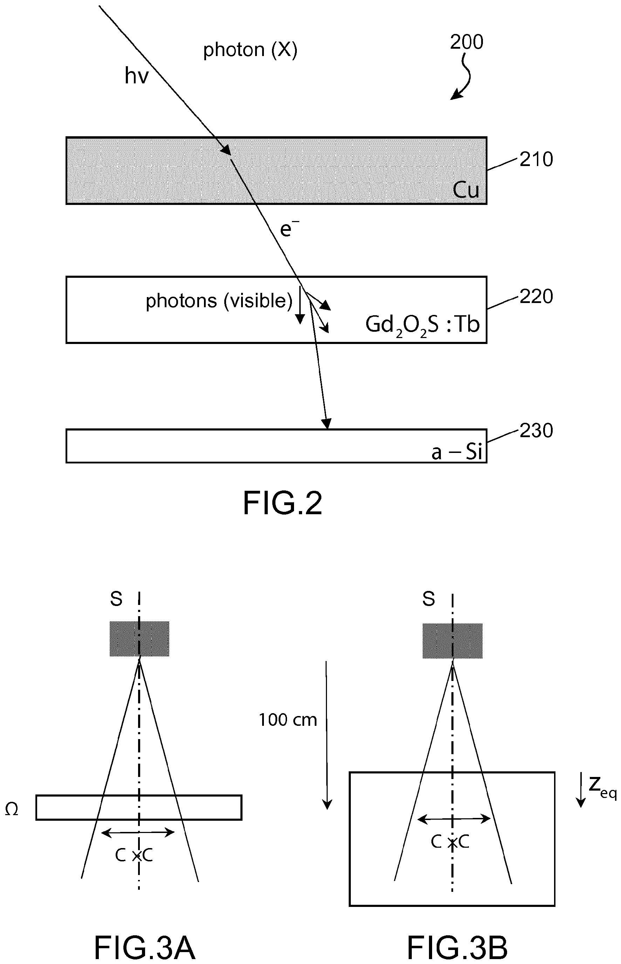

La Fig. 2 représente de manière schématique un détecteur à silicium amorphe à conversion indirecte, connu de l'état de la technique.

Ce détecteur comprend une plaque de métal, généralement du cuivre 210, dans laquelle les photons X incidents (photons primaires) cèdent une partie de l'énergie à des électrons par effet Compton. Ces électrons viennent frapper un scintillateur au phosphore ou un écran fluorescent, par exemple en oxysulfure de gadolinium dopé au terbium ($Gd_2O_2S{:}Tb$), 220, pour y générer des photons dans le domaine visible (photons secondaires).

Ces photons secondaires créent des paires électrons-trous dans une couche de silicium amorphe, 230, à laquelle est appliqué un champ électrique. Le courant électrique engendré par les paires électrons-trous est proportionnel au flux lumineux incident.

Le détecteur est pixellisé au sens où la couche de silicium amorphe comporte une matrice de photodiodes. Les charges générées dans le silicium amorphe sont lues par la matrice de transistors à effet de champ au moyen d'électrodes ligne et d'électrodes colonne. Chaque pixel fournit ainsi une valeur qui peut être numérisée et représentée par un niveau de gris. On obtient ainsi une matrice de valeurs numériques ou trame (encore dénommée *frame*).

La lecture des trames étant effectuée à une fréquence donnée (cadence trame) et les doses étant délivrées sous la forme d'impulsions calibrées se succédant avec une fréquence de récurrence donnée. Les trames peuvent être enregistrées une par une (mode dit continu) ou moyennées dans le temps (mode dit intégré). Dans ce dernier mode, on comprend qu'une image portale représente en fait une moyenne dans le temps des charges générées dans le silicium amorphe pendant une période d'exposition du faisceau. L'image portale est affichée sur l'imageur de l'EPID en niveaux de gris.

Toute la difficulté de la dosimétrie consiste alors à obtenir la dose délivrée (plus précisément la dose de photons arrivant sur le détecteur) ou image en dose à partir de l'image portale.

En pratique, la dose délivrée est reliée à une quantité d'unités moniteur ou UM. Une unité moniteur est une unité machine fixant la durée d'irradiation, pour une durée et une fréquence données des impulsions d'irradiation. Le système de radiothérapie est calibré de sorte qu'à une unité moniteur corresponde une dose déposée prédéterminée (généralement 1 cGy) dans un fantôme d'eau, à une profondeur de référence, $z_{ref}$, sur l'axe du faisceau, pour une taille de champ de 10x10 $cm^2$, la surface supérieure du fantôme d'eau étant située à une distance de référence (par exemple 100 cm) de la source de photons. Le protocole de calibration est décrit dans le rapport TRS398 de l'AIEA, Juin 2000. On rappelle qu'un Gray est une unité d'énergie déposée rapportée à la masse. L'énergie déposée à une profondeur $z$ est la différence entre l'énergie qui entre dans un petit volume de hauteur $dz$ à la profondeur $z$ et l'énergie qui sort de

$$D = \frac{dE}{\rho S dz}$$

ce petit volume. La dose à la profondeur $z$ est donnée par où $dE$ est la différence d'énergie précitée à la profondeur $z$ et $\rho S dz$ est la masse à laquelle elle est rapportée, $S$ étant la surface du champ et $\rho$ la masse volumique du matériau irradié. La référence à un fantôme d'eau en radiothérapie est justifiée en raison du fort pourcentage d'eau dans le corps humain.

Différentes méthodes ont été proposées pour convertir l'image portale en image de dosimétrie.

Une première méthode connue sous le terme modèle « CU » est décrite dans l'article de Van Esch et al. intitulé « The use of an a Si-based EPID for routine absolute dosimetry pre-treatment verification of dynamic IMRT fields » publié dans Radiotherapy and Oncology No 71 (2004), pp. 222-234. Cette méthode suppose une relation linéaire entre la dose, le niveau de gris dans l'image portale et le temps d'exposition, soit :

$$\hat{D} = a.N_g.T_{irrad} \qquad (1)$$

où $\hat{D}$ est la dose délivrée, estimée par la méthode, $N_g$ est le niveau de gris du pixel dans l'image et $T_{irrad}$ est le temps d'exposition au faisceau, $a$ est un facteur scalaire.

Une seconde méthode connue sous le terme modèle « frames » est décrite dans l'article de McDermott et al. intitulé « Dose response and ghosting effects of an amorphous silicon electronic portal imaging device » publié dans Med. Phys. Vol. 31, No 2, Février 2004. Cette méthode suppose une relation linéaire entre la dose délivrée, le niveau de gris dans l'image portale et le nombre total de trames intervenant dans l'image portale, soit :

$$\hat{D} = a.N_g.N_f + b \qquad (2)$$

où $\hat{D}$ est la dose délivrée, estimée par la méthode, $N_g$ est le niveau de gris du pixel dans l'image, $N_f$ est le nombre total de trames intervenant dans l'image portale, *a* est un facteur scalaire et *b* une valeur d'offset.

D'autres modèles ont été proposés, notamment dans l'article de P. Winkler et al. intitulé « Data response characteristics of an amorphous silicon EPID », publié dans Med. Phys., vol. 32, N° 10, Octobre 2005, pp. 3095-3105. Ce modèle est fondé sur une relation non linéaire entre le niveau de gris dans l'image portale et le nombre d'unités moniteur pendant une période trame, soit :

$$\frac{N_g}{UM} = a\ln(UM) + b \qquad\qquad (3)$$

où, $N_g$ est le niveau de gris du pixel dans l'image, $UM$ est le nombre d'unités moniteur, et *a, b* sont des constantes. En fait, comme expliqué dans l'article précité une correction de la dose délivrée doit encore être effectuée pour tenir compte de l'établissement du faisceau (*beam start-up*).

Enfin le document US-B-8130905 propose une méthode pour estimer la dose délivrée par un système de radiothérapie externe, à partir de l'image portale. Cette méthode comprend une étape de correction d'image au moyen d'une multiplication par une matrice de correction spécifique au faisceau, une étape de convolution avec un noyau de redistribution pour obtenir une dose équivalente dans l'eau à partir de la dose dans l'air, et enfin une étape de correction de la dose à partir d'une carte de calibration.

Toutefois, aucune de ces méthodes ne donne des résultats satisfaisants pour des faibles doses (doses délivrées au détecteur inférieures à un seuil de 40 cGy). Or, la multiplication des angles d'exposition dans les techniques de radiothérapie conformationnelle actuelles conduisent bien souvent à délivrer des doses inférieures au seuil précité pour une exposition donnée.

En outre, les valeurs de doses délivrées, obtenues à partir des images portales, ne sont généralement plus correctes dès lors que l'on s'éloigne de l'axe du faisceau. On fait alors appel à des formules de correction complexes comme décrit par exemple dans l'article de P. Winkler et al. intitulé « Implementation and validation of portal dosimetry with an amorphous silicon EPID in the energy range from 6 to 25 MV », publié dans Phys. Med. Biol., vol. 52, 2007, pp. N355-N365.

Le but de la présente invention est par conséquent de proposer une méthode de vérification de la dose délivrée par un système de radiothérapie externe équipé d'un imageur portal, qui ne présente pas les défauts précités, en particulier qui permette d'obtenir une estimation correcte de la dose y compris lorsque celle-ci est faible.

## EXPOSÉ DE L'INVENTION

La présente invention est définie par une méthode d'estimation de dose délivrée par un système de radiothérapie externe équipé d'un imageur portal, selon laquelle :

(a) on acquiert une image portale au moyen de l'imageur portal ;
(b) on détermine le niveau de gris, $N_g$, du pixel au centre de l'image portale, correspondant à l'intersection de l'imageur portal avec l'axe du faisceau d'irradiation ;
(c) on calcule la dose délivrée par le système à une profondeur de référence ($z_{ref}$) dans l'eau au moyen de

$$\frac{D}{D_{10}^{eq}} = \alpha + \beta\ln\left(\frac{N_g}{N_{g,10}}\right)$$ où $N_{g,10}$ est le niveau de gris du pixel au centre de l'image, obtenu pour une dose et des conditions d'irradiation de référence, $D_{10}^{eq}$ est une dose constante prédéterminée et $\alpha$, $\beta$ sont des coefficients préalablement déterminés dans une phase de calibration préalable.

Préalablement à l'étape (b) on élimine avantageusement de l'image portale le bruit de fond en lui soustrayant une image acquise en absence d'irradiation, pour obtenir une image portale débruitée.

De même, préalablement à l'étape (b), on peut corriger l'image débruitée en la débarrassant d'une composante due à la rétrodiffusion du faisceau par l'environnement de l'imageur portal.

La phase de calibration préalable comprend avantageusement :

- une étape d'acquisition d'une pluralité *K* d'images portales pour une pluralité de doses, $D^k$, *k* = 1,..., *K*, et dans des conditions d'irradiation de référence ;
- une étape d'élimination du bruit en soustrayant desdites images portales une image acquise en absence d'irradiation pour obtenir une pluralité $K$ d'images débruitées ;
- une étape de correction des images débruitées pour les débarrasser d'une composante due à la rétrodiffusion par l'environnement de l'imageur ;

- une étape de calcul des coefficients $\alpha$, $\beta$ à partir des valeurs $\dfrac{D^k}{D_{10}^{eq}}$ et $\ln\left(\dfrac{N_g^k}{N_{g,10}}\right)$, $k = 1,..., K.$

Les coefficients $\alpha$, $\beta$ sont avantageusement obtenus à partir des valeurs $\dfrac{D^k}{D_{10}^{eq}}$ et $\ln\left(\dfrac{N_g^k}{N_{g,10}}\right)$, $k = 1,..., K$ au moyen d'une régression linéaire.

Ladite phase de calibration préalable peut inclure en outre une phase de validation des coefficients $\alpha$, $\beta$ comprenant :

- une étape de mesure de distribution bidimensionnelle de dose délivrée à la profondeur de référence pour chacune des doses auxquelles les images portales ont été acquises, ladite étape de mesure fournissant une pluralité $K$ d'images en dose mesurées ;
- une étape de transformation des images portales acquises en images en dose, pour fournir une pluralité $K$ d'images en dose calculées;
- une étape de comparaison desdites images en dose mesurées avec lesdites images en dose calculées, les coefficients $\alpha$, $\beta$ étant validés en cas de concordance entre les premières et les secondes.

Selon, une variante chaque image portale $k = 1,..., K$ est transformée en une image en dose au moyen de la relation $\dfrac{\hat{D}^k(i, j)}{D_{10}^{eq}} = \alpha + \beta \ln\left(\dfrac{N_g^k(i, j)}{N_{g,10}}\right)$ où $N_g^k(i, j)$ est le niveau de gris du pixel $(i, j)$ de la $k$ ième image portale acquise.

Les images en dose mesurées et les images en dose calculées peuvent être ensuite comparées au moyen d'une analyse d'index $\gamma$.

L'invention concerne en outre une méthode de vérification de plan de traitement pour un système de radiothérapie externe équipé d'un imageur portal, le plan de traitement étant défini par une pluralité $M$ de distributions bidimensionnelles de dose à une profondeur de référence, chaque distribution bidimensionnelle étant associée à un angle d'incidence, une quantité d'unités moniteur et une conformation du faisceau d'irradiation, chaque distribution bidimensionnelle de dose donnant une image en dose de consigne, ladite méthode comprenant les étapes suivantes:

- on acquiert une image portale pour chaque angle incidence, quantité d'unités moniteur et conformation du faisceau d'irradiation, de manière à obtenir une même pluralité $M$ d'images portales acquises :
- on transforme les images portales acquises en images en dose pour fournir une pluralité $M$ d'images en dose calculées;
- on compare lesdites images en dose calculées aux images en dose de consigne.

Préalablement à l'étape de transformation, on élimine avantageusement le bruit de fond des images portales acquises en leur soustrayant une image acquise en absence d'irradiation.

De même, préalablement à l'étape de transformation, on peut corriger les images portales acquises ainsi débruitées, en les débarrassant d'une composante due à la rétrodiffusion du faisceau par l'environnement de l'imageur portal.

La comparaison entre les images en dose calculées et les images en dose de consigne peut être réalisée au moyen d'une analyse d'index $\gamma$.

La phase de calibration préalable peut comprendre là aussi :

- une étape d'acquisition d'une pluralité $K$ d'images portales pour une pluralité de doses, $D^k$, $k = 1,..., K$, et dans des conditions d'irradiation de référence ;
- une étape d'élimination du bruit en soustrayant desdites images portales une image acquise en absence d'irradiation pour obtenir une pluralité $K$ d'images débruitées ;
- une étape de correction des images débruitées pour les débarrasser d'une composante due à la rétrodiffusion par l'environnement de l'imageur ;

- une étape (840) de calcul des coefficients $\alpha$, $\beta$ à partir des valeurs $\dfrac{D^k}{D_{10}^{eq}}$ et $\ln\left(\dfrac{N_g^k}{N_{g,10}}\right)$, $k = 1,..., K.$

On peut avantageusement calculer les coefficients $\alpha$, $\beta$ à partir des valeurs $\dfrac{D^k}{D_{10}^{eq}}$ et $\ln\left(\dfrac{N_g^k}{N_{g,10}}\right)$, $k = 1,...,$ $K$ au moyen d'une régression linéaire.

## BRÈVE DESCRIPTION DES DESSINS

D'autres caractéristiques et avantages de l'invention apparaîtront à la lecture d'un mode de réalisation préférentiel de l'invention en référence aux figures jointes parmi lesquelles :

La Fig. 1 représente de manière schématique un système de radiothérapie externe équipé d'un imageur portal, connu de l'état de la technique ;
La Fig. 2 représente de manière schématique un exemple connu de détecteur utilisé dans le système de radiothérapie externe de la Fig. 1;
Les Figs 3A-3B illustrent la manière dont la profondeur équivalente est déterminée ;
Les Figs. 4A-4D représentent des étapes de modélisation permettant d'estimer la dose délivrée par un système de radiothérapie externe à partir d'une image portale ;
Les Figs. 5A-5C représentent respectivement la variation de l'inverse d'un facteur d'ouverture du collimateur à la profondeur équivalente, du rapport tissu maximum, et du facteur d'ouverture du collimateur à la profondeur de référence ;
La Fig. 6 représente de manière schématique l'ordinogramme d'une méthode d'estimation de la dose délivrée par un système de radiothérapie externe, selon un mode de réalisation de l'invention;
La Fig. 7 représente de manière schématique l'ordinogramme d'une méthode de vérification d'un plan de traitement par système de radiothérapie externe ;
La Fig. 8 représente de manière schématique l'ordinogramme d'une méthode de calibration pour la méthode d'estimation de dose délivrée selon la Fig. 6 ou la méthode de vérification d'un plan de traitement selon la Fig. 7 ;
Les Figs. 9 et 10 représentent des comparaisons par analyse d'index $\gamma$ entre des images en dose de consigne, issues d'un plan de traitement et des images en dose calculées à partir d'images portales.

## EXPOSÉ DÉTAILLÉ DE MODES DE RÉALISATION PARTICULIERS

On considérera dans la suite un système de radiothérapie externe équipé d'un imageur portal comme décrit dans la partie introductive. L'imageur portal peut être à conversion directe ou indirecte. Quel que soit le mode de conversion, les photons primaires ou secondaires génèrent des paires électrons-trous dans un détecteur à semi-conducteur pixellisé, par exemple un détecteur à semi-conducteur amorphe comme connu de l'art antérieur.

La méthode d'estimation de la dose délivrée par le système utilise l'image portale fournie par l'imageur. Par dose, on entend dans la suite l'énergie déposée par le faisceau dans l'eau par unité de masse, à une profondeur de référence donnée, $z_{ref}$. Conventionnellement cette profondeur de référence est choisie égale à 5 ou 10cm, en fonction de la qualité du faisceau, à compter de la surface de l'eau.

L'image portale est constituée par la moyenne de $N_f$ trames successives sur la durée de l'exposition. De manière similaire, la dose délivrée en un point est la somme des doses élémentaires délivrées en ce point pendant la durée de chacune des trames. On peut supposer que la dose par trame est constante et égale à une dose moyenne $\tilde{D}$.

La méthode d'estimation de la dose délivrée repose, selon la présente invention, sur un modèle original reliant le niveau de gris de l'image portale avec la dose par trame. Plus précisément, il a pu être montré que le niveau de gris d'un pixel dans une image portale était relié à la dose par trame, au moyen de la relation suivante :

$$\frac{N_g}{N_{g,10}} = A \exp\left(B \frac{\tilde{D}}{\tilde{D}_{10}^{eq}}\right) \qquad (4)$$

où $N_g$ est le niveau de gris dans l'image portale, $\tilde{D}$ est la dose délivrée par le système de radiothérapie pendant la durée d'une trame, $A$ est une constante représentant la réponse du détecteur pour une dose nulle et $B$ est un paramètre dépendant de l'énergie des photons incidents, représentant l'atténuation des photons dans le détecteur. $N_{g,10}$ et $\tilde{D}_{10}^{eq}$ représentent respectivement le niveau de gris au centre de l'image portale et la dose correspondante en ce point, dans des conditions de référence. Dans la suite, on raisonnera par trame et l'on utilisera par souci de simplification les notations $D$ et $D_{10}^{eq}$.

De manière générale, la dose déposée à une profondeur donnée comprend une première composante, dite composante primaire, résultant de photons issus de la source et interagissant pour la première fois avec le matériau, et une composante, dite composante de diffusion, résultant de photons ayant déjà interagi une première fois avec le matériau. La composante diffusée dépend notamment de la taille du champ d'irradiation, de sorte que la dose déposée à une profondeur donnée ne varie pas de manière simplement linéaire avec la fluence à l'entrée du matériau.

En revanche, il a pu être montré qu'il existait une profondeur d'eau, dite profondeur équivalente et notée $z_{eq}$, pour laquelle la réponse de l'imageur portal était proportionnelle à la dose déposée à cette profondeur et, ce, quelle que soit la taille du champ d'irradiation.

Cette situation d'équivalence a été illustrée en Figs. 3A et 3B.

On a représenté schématiquement en Fig. 3A la source de photons X émettant un faisceau F arrivant sur le détecteur $\Omega$. On suppose que le champ d'irradiation du faisceau est de taille carrée et de dimension $C \times C$ (exprimée en cm$^2$). Le niveau de gris au centre de l'image portale est noté $N_g$.

En Fig. 3B on a représenté la même source S avec le même faisceau de photons qu'en Fig. 3A. Le faisceau est ici incident sur un fantôme d'eau. On a également la profondeur d'eau équivalente $z_{eq}$ au sens défini plus haut. La taille du champ d'irradiation dans le plan à la profondeur d'eau équivalente est de $C \times C$. Ce plan est choisi à une distance de référence (ici 100 cm) de la source.

La linéarité de la réponse de l'imageur portale vis-à-vis de la dose délivrée se traduit par :

$$\frac{N_g}{N_{g,10}} = \frac{D_c^{eq}}{D_{10}^{eq}} \qquad (5)$$

où $N_{g,10}$ est le niveau de gris obtenu pour les conditions de calibration, à savoir pour un champ de taille 10x10 cm$^2$ et une dose par trame correspondant à 100 UM.

Les mesures montrent que la profondeur équivalente $z_{eq}$ varie avec l'énergie des photons incidents mais reste toujours inférieure à la profondeur d'équilibre $z_V$ dans l'eau. Il en résulte que la profondeur équivalente ne peut servir de profondeur de référence.

L'idée à la base de l'invention est de déduire la réponse de l'imageur portal en fonction de la dose délivrée à une profondeur de référence, $z_{ref}$, à partir de la réponse de cet imageur en fonction de la dose délivrée à la profondeur équivalente, $z_{eq}$.

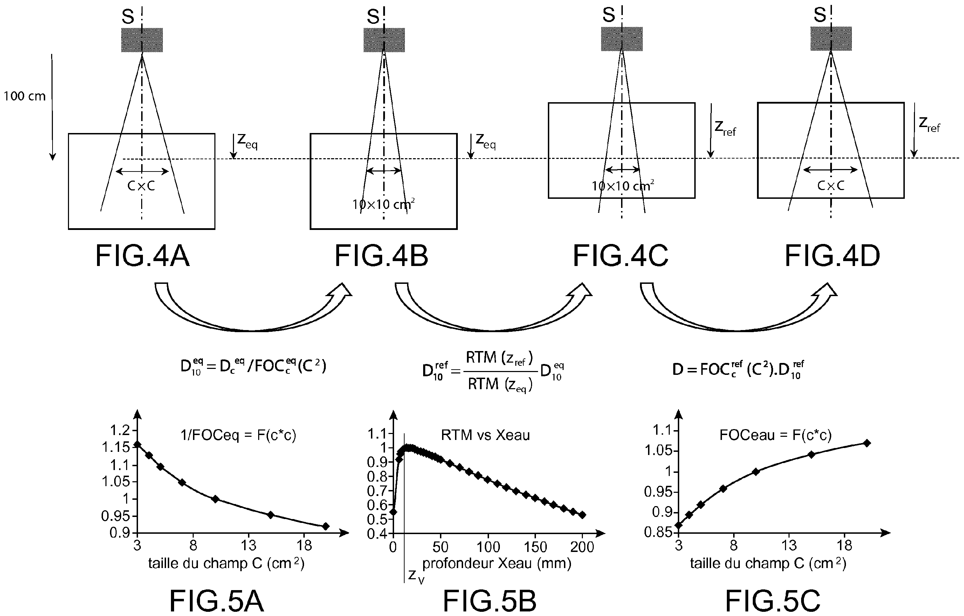

Cette déduction peut être obtenue en procédant par les étapes de modélisation représentées en Figs. 4A à 4D.

La Fig. 4A reprend simplement la Fig. 3B, autrement dit un fantôme d'eau dont la surface supérieure est située à $100cm\text{-}z_{eq}$ de la source de photons avec une géométrie de faisceau telle que la taille du champ d'irradiation à la profondeur équivalente est de $C \times C$ cm$^2$.

La Fig. 4B représente la même situation que celle de la Fig. 4A à la différence près que la taille du champ est ici la taille du champ de référence $10 \times 10$ cm$^2$. Le faisceau présente par conséquent une divergence différente.

Si l'on note $D_c^{eq}$ la dose déposée dans l'eau à la profondeur équivalente $z_{eq}$ pour un faisceau de champ carré de dimension $C \times C$ cm$^2$, et $D_{10}^{eq}$ la dose déposée dans l'eau à la profondeur équivalente mais pour un champ d'irradiation carré de taille $10 \times 10$ cm$^2$, on a la relation :

$$D_c^{eq} = FOC_c^{eq}(C^2).D_{10}^{eq} \qquad (6)$$

où $FOC_c^{eq}$ est le facteur d'ouverture du collimateur mesuré dans l'eau à la profondeur équivalente $z_{eq}$. De manière équivalente :

$$D_{10}^{eq} = D_c^{eq} / FOC_c^{eq}(C^2) \qquad (7)$$

La variation de l'inverse du facteur d'ouverture du collimateur, $1\!\!/ FOC_c^{eq}(C^2)$ en fonction de la taille du champ $C^2$ est donnée en Fig. 5A.

On a représenté en Fig. 4C la même situation qu'à la Fig. 4B à la différence près que la profondeur à laquelle on détermine la dose déposée dans l'eau n'est plus la profondeur équivalente $z_{eq}$ mais la profondeur de référence $z_{ref}$. Le rapport entre la dose déposée dans l'eau à une profondeur quelconque et la dose maximale $D_{max}$ est dénommé en dosimétrie « rapport tissu maximum » (*RTM*).

Le rapport *RTM* varie en fonction de la profondeur dans l'eau selon la courbe représentée en Fig. 5B. Cette courbe présente une première région, dite région de mise en équilibre électronique (*build up region*) jusqu'à une profondeur d'équilibre $z_V$, à laquelle la dose déposée atteint son maximum, puis une seconde région, dite région d'équilibre, dans laquelle la dose déposée décroît de manière exponentielle.

Autrement dit, le rapport tissu maximum n'est que la dose déposée, normalisée par la dose maximale. On a par conséquent la relation :

$$D_{10}^{eq} = RTM(z_{eq}).D_{max} \qquad (8)$$

et, de manière similaire :

$$D_{10}^{ref} = RTM(z_{ref}).D_{max} \qquad (8')$$

où $D_{10}^{ref}$ est la dose déposée à la profondeur de référence dans l'eau, pour les conditions d'irradiation de référence (distance de la source au plan de 100cm et taille de champ d'irradiation de $10 \times 10$ cm$^2$ dans ce plan.

Il en résulte:

$$D_{10}^{ref} = \frac{RTM(z_{ref})}{RTM(z_{eq})}.D_{10}^{eq} \qquad (9)$$

La Fig. 4D représente la même situation que celle de la Fig. 4C à la différence près que le faisceau présente désormais une taille de champ $C^2$ à la profondeur de référence, autrement dit la même taille de champ que celle du faisceau en Fig. 4A.

On rappelle que la dose déposée à la profondeur de référence dans le fantôme d'eau est la dose $D$ que l'on souhaite estimer. Celle-ci est reliée à la dose $D_{10}^{ref}$ par la relation :

$$D = FOC_c^{ref}(C^2).D_{10}^{ref} \qquad (10)$$

où $FOC_c^{ref}$ est le facteur d'ouverture du collimateur, mesuré dans l'eau à la profondeur de référence $z_{ref}$.

On déduit des équations (7), (9) et (10) que :

$$D = D_c^{eq}.\frac{RTM(z_{ref})}{RTM(z_{eq})}.\frac{FOC_c^{ref}(C^2)}{FOC_c^{eq}(C^2)} \qquad (11)$$

La variation du facteur d'ouverture du collimateur $FOC_c^{ref}$ en fonction de la taille du champ $C^2$ a été représentée en Fig. 5C.

On a pu montrer que le facteur d'ouverture du collimateur à la profondeur de référence était lié au facteur d'ouverture du collimateur à la profondeur équivalente, selon une loi logarithmique, autrement dit :

$$FOC_c^{ref} = \alpha' + \beta' \ln\left(FOC_c^{eq}\right) \qquad (12)$$

où les constantes $\alpha'$, $\beta'$ ne dépendent que de l'énergie des photons incidents.

Cette relation peut s'exprimer, compte tenu de (6), (9) et (10) :

$$\frac{D}{D_{10}^{eq}} = \alpha + \beta \ln\left(\frac{D_c^{eq}}{D_{10}^{eq}}\right) \qquad (13)$$

où

$$\alpha = \alpha' \frac{RTM\left(z_{ref}\right)}{RTM\left(z_{eq}\right)} \text{ et } \beta = \beta' \frac{RTM\left(z_{ref}\right)}{RTM\left(z_{eq}\right)}.$$

Etant donné que le niveau de gris au centre de l'image portale est proportionnel à la dose mesurée à la profondeur équivalente, on a d'après (5):

$$\frac{D_c^{eq}}{D_{10}^{eq}} = \frac{N_g}{N_{g,10}} \qquad (14)$$

où $N_{g,10}$ est le niveau de gris obtenu pour les conditions de calibration, à savoir pour un champ de taille 10x10 cm$^2$ et une dose par trame correspondant à 100 UM.

Il résulte des équations (13) et (14) que :

$$\frac{D}{D_{10}^{eq}} = \alpha + \beta \ln\left(\frac{N_g}{N_{g,10}}\right) \qquad (15)$$

ou, de manière équivalente :

$$\frac{N_g}{N_{g,10}} = A \exp\left[B \frac{D}{D_{10}^{eq}}\right] \qquad (16)$$

avec

$$A = \exp\left(-\alpha/\beta\right) \text{ et } B = 1/\beta.$$

On comprendra ainsi qu'il est possible, à partir de l'expression (15), de convertir une image portale en une image en dose délivrée. Cette conversion ne nécessite pas de convolution par un noyau ou de corrections complexes en fonction de la collimation du faisceau.

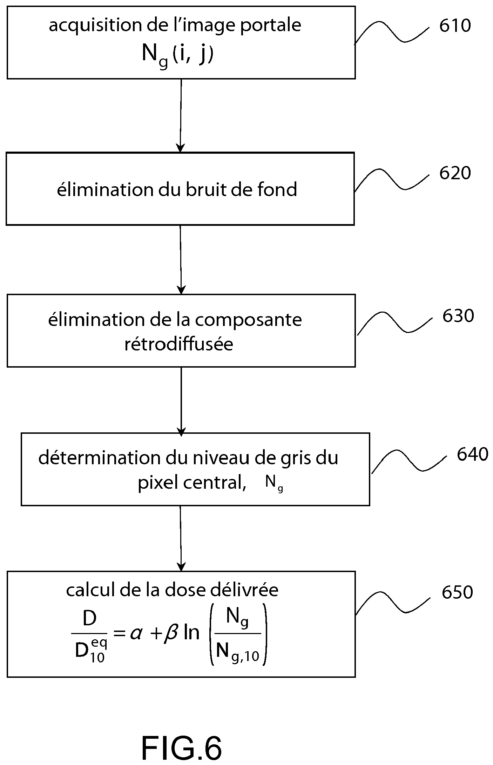

Plus précisément, la Fig. 6 représente de manière schématique la méthode d'estimation de dose délivrée par un système de radiothérapie externe, selon un mode de réalisation de l'invention.

A l'étape 610, on fait l'acquisition d'une image portale en niveaux de gris au moyen de l'imageur portal. L'image peut être représentée par une matrice $\mathbf{N_g}$ dont les éléments sont les niveaux de gris $N_g(i, j)$ des pixels de l'image.

A l'étape 620, on élimine de l'image portale l'image du bruit de fond, dite aussi image dans les conditions d'obscurité ou DFI (*Dark Field Image*). Cette image est obtenue en faisant une acquisition par l'imageur portal en absence d'irradiation. Si l'on note $\mathbf{N}_g^0$ la matrice dont les éléments sont les niveaux de gris des pixels dans les conditions d'obscurité, l'opération d'élimination du bruit de fond consiste simplement à calculer la matrice $\mathbf{N_g} - \mathbf{N}_g^0$, dite matrice débruitée.

A l'étape 630, on corrige l'image ainsi débruitée pour tenir compte du rayonnement rétro-diffusé par le matériel du système de radiothérapie, en particulier par le bras mécanique. La correction est effectuée en débarassant l'image de la composante due à la rétro-diffusion. Cette correction peut être réalisée comme décrit par exemple dans l'article de B.W. King et al. intitulé « A method for removing arm backscatter from EPID images », publié dans Med. Phys., vol. 40, n° 7, Juillet 2013, pp. 071703-1 à 071703-9.

A l'étape 640, on détermine le niveau de gris, $N_g$, du pixel au centre de l'image portale, c'est-à-dire au point d'intersection de l'image portale avec l'axe du faisceau.

A l'étape 650, on calcule la dose délivrée, $D$, au moyen de l'expression (15) à partir du niveau de gris $N_g$, les paramètres $\alpha, \beta$ ayant été obtenus au moyen d'une procédure préalable de calibration décrite plus loin.

On notera que si les étapes 620 et 630 sont nécessaires pour obtenir une estimation précise de la dose délivrée, elles peuvent être omises lorsque l'on peut se contenter d'une estimation plus grossière.

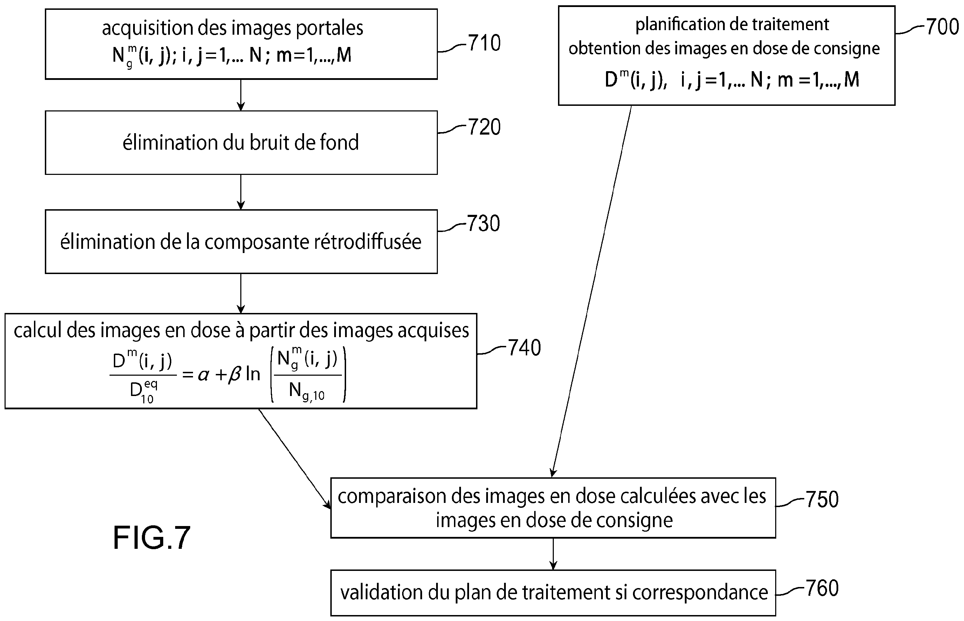

La Fig. 7 représente de manière schématique une méthode de vérification de la dose délivrée par un système de radiothérapie externe, selon un mode de réalisation de l'invention.

Cette méthode trouve particulièrement à s'appliquer dans le cadre d'une planification de traitement de radiothérapie.

On a représenté en 700 une étape de planification d'un traitement par radiothérapie externe. Cette étape consiste à déterminer une pluralité $M$ d'angles d'incidence du faisceau et pour chaque angle d'incidence une conformation du faisceau (c'est-à-dire la disposition des lames du collimateur), une distance de la source à la table de traitement et une quantité d'unités moniteur. Autrement dit, pour chaque angle d'incidence, on détermine une matrice bidimensionnelle de dose, $\overline{D}^m(i,j), i, j = 1,..., N,$ représentant une cartographie de la dose à délivrer au patient. Cette matrice bidimensionnelle de dose peut être représentée comme une image $I^m$ de dose de consigne dont chaque pixel de coordonnées $(i, j)$ a pour niveau de gris la dose $\overline{D}^m(i, j)$.

Parallèlement, en 710, on fait l'acquisition, en absence de patient, des images portales du faisceau au moyen du détecteur EPID, sous les différents angles d'incidence, selon les différentes conformations et avec le nombre d'UM spécifiés dans le plan de traitement. Chaque image portale, $m = 1,..., M$ est définie par les niveaux de gris de ses pixels, soit $N_g^m(i, j), i, j = 1,..., N$.

Chacune de ces images peut être corrigée, en 720, par élimination du bruit de fond, comme décrit précédemment, et, en 730, par élimination du rayonnement rétrodiffusé par le matériel du système de radiothérapie.

A l'étape 740, on transforme l'image portale en image en dose délivrée au moyen de l'expression :

$$\frac{D^m(i, j)}{D_{10}^{eq}} = \alpha + \beta \ln\left(\frac{N_g^m(i, j)}{N_{g,10}}\right) \qquad (17)$$

A l'étape 750, on compare chaque image en dose calculées $D_m(i, j), m = 1,..., M,$ à l'étape précédente avec les images en dose de consigne $\overline{D}_m(i, j), m = 1,..., M,$ fournies par le plan de traitement. La comparaison des images calculées avec les images de consigne est avantageusement effectuée au moyen d'une analyse d'index $\gamma$, connue dans le domaine de la dosimétrie et rappelée plus loin.

A l'étape 760, si les images en doses calculées et les images en dose de consigne sont concordantes au sens d'une analyse d'index $\gamma$, on valide le plan de traitement.

L'homme du métier comprendra qu'alternativement, on pourra convertir les images en dose de consigne en images portales de consigne au moyen de la relation (16), et comparer les images portales acquises avec les images portales de consigne. La comparaison pourra être également faite au moyen d'une analyse d'index $\gamma$.

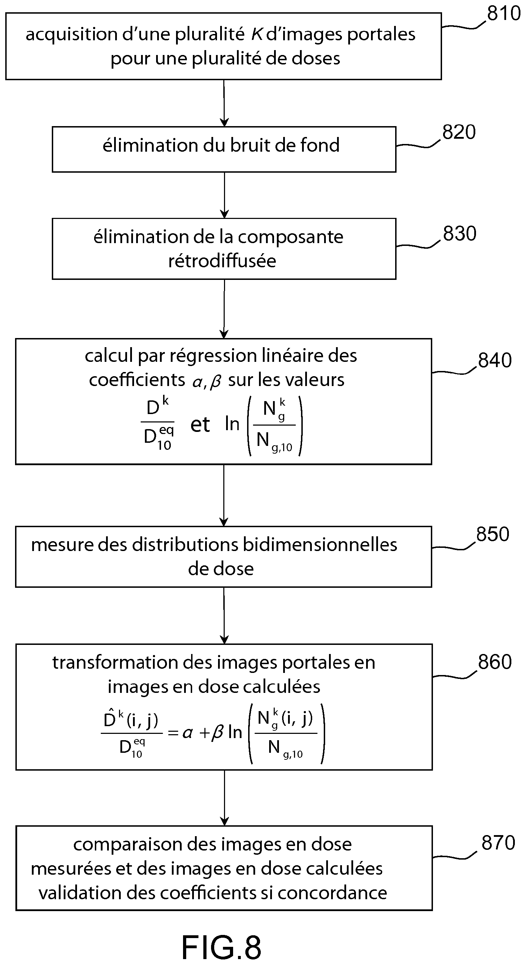

La Fig. 8 représente de manière schématique une méthode de calibration pour la méthode d'estimation de dose délivrée de la Fig. 6 ou la méthode de vérification de plan de traitement de la Fig. 7.

A la première étape 810, on acquiert une pluralité K d'images au moyen du détecteur EPID du système de radiothérapie externe. Les images sont acquises pour une pluralité de doses, par exemple des doses variant de 2 à 400 UM. L'acquisition se fait en absence de patient, dans des conditions d'irradiation de référence, c'est-à-dire avec un faisceau conformé pour présenter un champ carré de taille 10x10 cm$^2$ sur le détecteur. Le détecteur est placé à une distance de 100 cm de la source de photons X.

A l'étape 820, on effectue une première correction des images acquises en éliminant le bruit de fond comme expliqué plus haut, en relation avec l'étape 620 de la Fig. 6.

A l'étape 830, on effectue une seconde correction des images en éliminant des images ainsi débruitées la contribution du rayonnement rétrodiffusé par l'environnement (notamment par le bras mécanique du système). Cette correction est effectuée comme expliqué plus haut en relation avec l'étape 630 de la Fig. 6.

A l'étape 840, on calcule les coefficients $\alpha, \beta$ en effectuant une régression linéaire sur les points de coordonnées

$\dfrac{D^k}{D_{10}^{eq}}$ et $\ln\left(\dfrac{N_g^k}{N_{g,10}}\right)$, $k = 1,..., K$ où $D^k$ est la dose par trame ayant servi à générer l'image $k$, $N_g^k$ est le niveau de gris du pixel au centre de l'image $k = 1,..., K$ (autrement dit sur l'axe du faisceau), $N_{g,10}$ représente le niveau de gris au centre de l'image pour une dose par trame correspondant à un faisceau de 10x10cm$^2$ et 100UM.

Optionnellement, on pourra vérifier que les coefficients $\alpha, \beta$ sont bien corrects à l'aide des étapes 850-870.

Plus précisément, à l'étape 850, on mesure, à l'aide d'une chambre d'ionisation, la distribution bidimensionnelle de la dose délivrée dans l'eau (ou simplement le profil transversal en dose dans un plan de symétrie du faisceau) à la profondeur de référence. Cette distribution peut être mesurée pour chacune des valeurs de dose à laquelle les images ont été obtenues à l'étape 810. On obtient ainsi, par la mesure, $K$ distributions bidimensionnelles en dose. Ces $K$ distributions bidimensionnelles peuvent être considérées comme des images en dose mesurées, $D^k(i, j)$ ; $i, j = 1,..., N$ ; $k = 1,.., K$.

A l'étape 860, on transforme les images portales acquises en images en dose calculées au moyen de la relation :

$$\frac{\hat{D}^k(i, j)}{D_{10}^{eq}} = \alpha + \beta \ln\left(\frac{N_g^k(i, j)}{N_{g,10}}\right) \tag{18}$$

où $N_g^k(i, j)$ est le niveau de gris du pixel $(i, j)$ de la $k$ ème image portale acquise.

A l'étape 870, on compare les images en dose calculées à l'étape précédente avec les images en dose mesurées à l'étape 850. Cette comparaison est réalisée sur la base d'une analyse d'index $\gamma$ comme indiqué précédemment.

Lorsque ces images concordent, on peut conclure à la validité des coefficients $\alpha, \beta$.

La méthode de comparaison de deux images sur la base de l'index $\gamma$ est décrite dans l'article de D.A. Low et al. intitulé « A technique for the quantitative evaluation of dose distributions » publié dans la revue Med. Phys., Vol. 25, N° 5, pp. 656-661, et est rappelée ci-après.

La méthode de comparaison sur la base d'une analyse d'index $\gamma$ permet de comparer une image par rapport à une image de référence. Elles peuvent être représentées respectivement par des variables bidimensionnelles $I(x, y)$ (image à comparer) et $I_r(x, y)$ (image de référence), ces variables pouvant être par exemple des valeurs de dose ou des niveaux de gris d'images portales.

Les variables bidimensionnelles $I(x, y)$ et $I_r(x, y)$ peuvent être représentées par des nappes dans un espace de dimension 3, à chaque point de coordonnées $x, y$ étant associé un point d'ordonnée $I(x, y)$, resp. $I_r(x, y)$. On appellera dans la suite nappe de référence, $\Lambda_r$, la nappe de l'image de référence et nappe courante, $\Lambda$, la nappe de l'image à comparer.

A chaque point $P$ de $\Lambda_r$, de coordonnées $(x_P y_P, z_p = I_r(x_p, y_p))$ de la nappe de référence, on positionne un ellipsoïde de révolution de rayon $\delta r_{max}$ et de hauteur $\delta I_{max}$. On détermine alors s'il existe un point $Q$ de la nappe courante présent dans l'ellipsoïde.

Plus précisément, l'index $\gamma$ évalué au point de coordonnées $(x_P, y_P)$ est donné par :

$$\gamma\left(x_P, y_P\right) = \min_{Q \in \Lambda}\left[\sqrt{\left(\frac{\delta r}{\delta r_{max}}\right)^2 + \left(\frac{\delta I}{\delta I_{max}}\right)^2}\right] \tag{19}$$

où

$$\delta I = \left| I(x_Q, y_Q) - I_r(x_P, y_P) \right| \text{ et } \delta r = \sqrt{\left( x_Q - x_P \right)^2 + \left( y_Q - y_P \right)^2} \, .$$

Une valeur d'index $\gamma$ inférieure à 1 indique une concordance entre les deux images au point considéré.

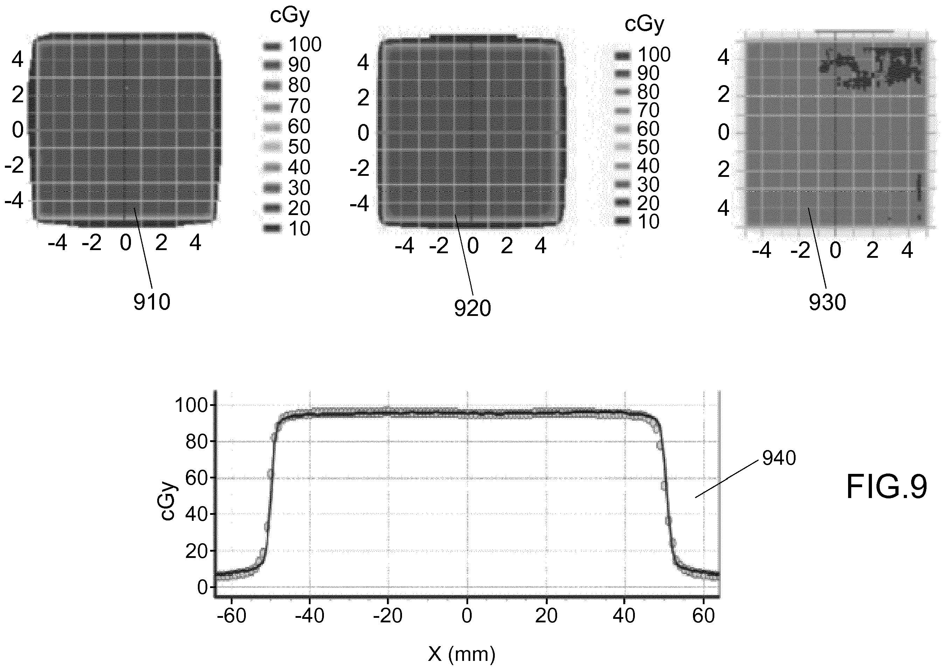

La Fig. 9 illustre une comparaison, par analyse en index $\gamma$, entre une image en dose de consigne 910, issue d'un plan de traitement, et une image en dose, calculée à partir d'une image portale, 920. La dose de consigne est ici uniforme dans le champ d'irradiation de taille 10x10 cm$^2$, pour 100 UM. L'index $\gamma$ est représenté en 930. En 940, on a représenté les profils selon l'axe OX de l'image en dose de consigne (trait continu) et de l'image en dose calculée (cercles). On remarque une bonne correspondance entre les deux profils. Seule une faible partie de l'image calculée présente une légère distorsion par rapport à l'image de consigne, comme le montre l'image d'index $\gamma$, 930 (partie en haut à droite de l'image). Cette légère distorsion provient ici de l'absence de correction de la composante rétrodiffusée.

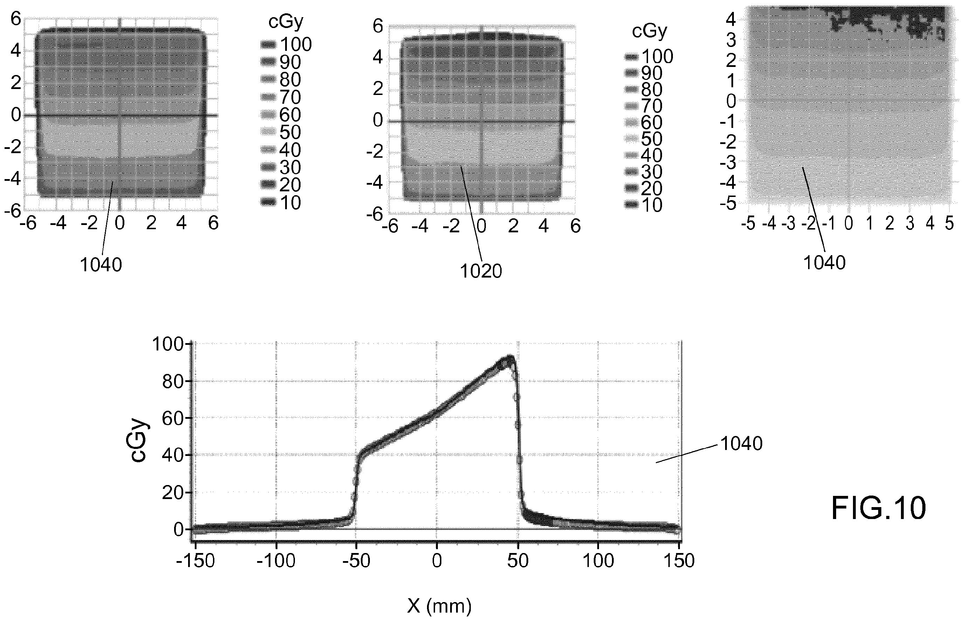

La Fig. 10 illustre une comparaison, par analyse en index $\gamma$, entre une image en dose de consigne 1010, issue d'un plan de traitement, et une image en dose, calculée à partir d'une image portale, 1020. La dose de consigne présente ici un gradient dans le champ d'irradiation (100 UM, champ d'irradiation de taille 10x10 cm$^2$ et filtre en coin orienté à 60° par rapport à l'axe du faisceau). L'index $\gamma$ est représenté en 1030. En 1040, on a représenté les profils selon l'axe vertical de l'image en dose de consigne (trait continu) et de l'image en dose calculée (cercles). On remarque une très bonne correspondance entre les deux profils. Seule une faible partie de l'image calculée présente une légère distorsion par rapport à l'image de consigne, comme le montre l'image d'index $\gamma$, 1030 (partie en haut de l'image). Comme précédemment, cette légère distorsion provient ici de l'absence de correction de la composante rétrodiffusée.

**Revendications**

1. Méthode d'estimation de dose délivrée par un système de radiothérapie externe équipé d'un imageur portal, dans laquelle

   (a) on acquiert (610) une image portale au moyen de l'imageur portal ;
   ladite méthode étant **caractérisée en ce que**:
   (b) on détermine (640) le niveau de gris, $N_g$, du pixel au centre de l'image portale, correspondant à l'intersection de l'imageur portal avec l'axe du faisceau d'irradiation ;
   (c) on calcule (650) la dose délivrée par le système à une profondeur de référence ($z_{ref}$) dans l'eau au moyen

   $$\frac{D}{D_{10}^{eq}} = \alpha + \beta \ln\left( \frac{N_g}{N_{g,10}} \right)$$

   de où $N_{g,10}$ est le niveau de gris du pixel au centre de l'image, obtenu pour une dose et des conditions d'irradiation de référence, $D_{10}^{eq}$ est une dose constante prédéterminée et $\alpha$, $\beta$ sont des coefficients préalablement déterminés dans une phase de calibration préalable.

2. Méthode d'estimation de dose selon la revendication 1, **caractérisée en ce que**, préalablement à l'étape (b) on élimine (620) de l'image portale le bruit de fond en lui soustrayant une image acquise en absence d'irradiation, pour obtenir une image portale débruitée.

3. Méthode d'estimation de dose selon la revendication 2, **caractérisée en ce que**, préalablement à l'étape (b), on corrige (630) l'image débruitée en la débarrassant d'une composante due à la rétrodiffusion du faisceau par l'environnement de l'imageur portal.

4. Méthode d'estimation de dose selon la revendication 3, **caractérisée en ce que** ladite phase de calibration préalable comprend :

   - une étape (810) d'acquisition d'une pluralité $K$ d'images portales pour une pluralité de doses, $D^k$, $k = 1,..., K$, et dans des conditions d'irradiation de référence ;
   - une étape (820) d'élimination du bruit en soustrayant desdites images portales une image acquise en absence d'irradiation pour obtenir une pluralité $K$ d'images débruitées ;
   - une étape (830) de correction des images débruitées pour les débarrasser d'une composante due à la rétrodiffusion par l'environnement de l'imageur ;

- une étape (840) de calcul des coefficients $\alpha$, $\beta$ à partir des valeurs $\dfrac{D^k}{D_{10}^{eq}}$ et $\ln\left(\dfrac{N_g^k}{N_{g,10}}\right)$, $k = 1,..., K$.

5. Méthode d'estimation de dose selon la revendication 4, **caractérisée en ce que** les coefficients $\alpha$, $\beta$ sont obtenus à partir des valeurs $\dfrac{D^k}{D_{10}^{eq}}$ et $\ln\left(\dfrac{N_g^k}{N_{g,10}}\right)$, $k = 1,..., K$ au moyen d'une régression linéaire.

6. Méthode d'estimation de dose selon la revendication 5, **caractérisée en ce que** ladite phase de calibration préalable inclut en outre une phase de validation des coefficients $\alpha$, $\beta$ comprenant :

   - une étape (850) de mesure de distribution bidimensionnelle de dose délivrée à la profondeur de référence pour chacune des doses auxquelles les images portales ont été acquises, ladite étape de mesure fournissant une pluralité $K$ d'images en dose mesurées ;
   - une étape (860) de transformation des images portales acquises en images en dose, pour fournir une pluralité $K$ d'images en dose calculées ;
   - une étape (870) de comparaison desdites images en dose mesurées avec lesdites images en dose calculées, les coefficients $\alpha$, $\beta$ étant validés en cas de concordance entre les premières et les secondes.

7. Méthode d'estimation de dose selon la revendication 6, **caractérisée en ce que** chaque image portale $k = 1,..., K$ est transformée en une image en dose au moyen de la relation $\dfrac{\hat{D}^k(i,j)}{D_{10}^{eq}} = \alpha + \beta \ln\left(\dfrac{N_g^k(i,j)}{N_{g,10}}\right)$ où $N_g^k(i,j)$ est le niveau de gris du pixel $(i, j)$ de la $k$ ième image portale acquise.

8. Méthode d'estimation de dose selon la revendication 6, **caractérisée en ce que** les images en dose mesurées et les images en dose calculées sont comparées au moyen d'une analyse d'index $\gamma$.

9. Méthode de vérification de plan de traitement pour un système de radiothérapie externe équipé d'un imageur portal, le plan de traitement étant défini (700) par une pluralité $M$ de distributions bidimensionnelles de dose à une profondeur de référence, chaque distribution bidimensionnelle étant associée à un angle d'incidence, une quantité d'unités moniteur et une conformation du faisceau d'irradiation, chaque distribution bidimensionnelle de dose donnant une image en dose de consigne, dans laquelle :

   - on acquiert une image portale pour chaque angle incidence, quantité d'unités moniteur et conformation du faisceau d'irradiation, de manière à obtenir une même pluralité $M$ d'images portales acquises :
   ladite méthode étant **caractérisée en ce que**
   - on transforme les images portales acquises en images en dose pour fournir une pluralité $M$ d'images en dose calculées, ladite transformation étant réalisée au moyen de l'expression $\dfrac{D^m(i,j)}{D_{10}^{eq}} = \alpha + \beta \ln\left(\dfrac{N_g^m(i,j)}{N_{g,10}}\right)$ où $D^m(i,j)$ est le niveau de gris du pixel de coordonnées $(i, j)$ de l'image en dose $m = 1,..., M$ et $N_g^m(i,j)$ le niveau de gris du pixel de coordonnées $(i, j)$ de l'image portale correspondante, $N_{g,10}$ est le niveau de gris du pixel au centre de l'image, obtenu pour une dose et des conditions d'irradiation de référence, $D_{10}^{eq}$ est une dose constante prédéterminée et $\alpha$, $\beta$ sont des coefficients préalablement déterminés dans une phase de calibration préalable ;
   - on compare lesdites images en dose calculées aux images en dose de consigne.

10. Méthode de vérification de plan de traitement selon la revendication 9, **caractérisée en ce que**, préalablement à l'étape de transformation, on élimine (720) des images portales acquises le bruit de fond en leur soustrayant une image acquise en absence d'irradiation.

**11.** Méthode de vérification de plan de traitement selon la revendication 10, **caractérisée en ce que**, préalablement à l'étape de transformation, on corrige (730) les images portales acquises ainsi débruitées, en les débarrassant d'une composante due à la rétrodiffusion du faisceau par l'environnement de l'imageur portal.

**12.** Méthode de vérification de plan de traitement selon la revendication 9, **caractérisée en ce que** la comparaison entre les images en dose calculées et les images en dose de consigne est réalisée au moyen d'une analyse d'index $\gamma$.

**13.** Méthode de vérification de plan de traitement selon l'une des revendications 9 à 12, **caractérisée en ce que** la phase de calibration préalable comprend :

- une étape (810) d'acquisition d'une pluralité $K$ d'images portales pour une pluralité de doses, $D^k, k = 1,..., K$, et dans des conditions d'irradiation de référence ;
- une étape (820) d'élimination du bruit en soustrayant desdites images portales une image acquise en absence d'irradiation pour obtenir une pluralité $K$ d'images débruitées ;
- une étape (830) de correction des images débruitées pour les débarrasser d'une composante due à la rétro-diffusion par l'environnement de l'imageur ;

- une étape (840) de calcul des coefficients $\alpha, \beta$ à partir des valeurs $\dfrac{D^k}{D_{10}^{eq}}$ et $\ln\left(\dfrac{N_g^k}{N_{g,10}}\right)$, $k = 1,..., K$.

**14.** Méthode de vérification de plan de traitement selon la revendication 13, **caractérisée en ce que** l'on calcule les coefficients $\alpha, \beta$ à partir des valeurs $\dfrac{D^k}{D_{10}^{eq}}$ et $\ln\left(\dfrac{N_g^k}{N_{g,10}}\right)$, $k = 1,..., K$ au moyen d'une régression linéaire.

**Patentansprüche**

**1.** Verfahren zum Abschätzen einer von einem externen Strahlungstherapiesystem abgegebenen Dosis, das mit einem Portalbildgeber ausgestattet ist, wobei

(a) mittels des Portalbildgebers ein Portalbild erfasst wird (610);
wobei das Verfahren **dadurch gekennzeichnet ist, dass**
(b) die Graustufe $N_g$ des Pixels in der Mitte des Portalbildes bestimmt wird (640), das dem Schnittpunkt des Portalbildgebers mit der Achse des Bestrahlungsbündels entspricht;
(c) die von dem System abgegebene Dosis bei einer Referenztiefe ($z_{ref}$) im Wasser berechnet wird (650) durch

$$\dfrac{D}{D_{10}^{eq}} = \alpha + \beta \ln\left(\dfrac{N_g}{N_{g,10}}\right),$$ wobei $N_{g,10}$ die Graustufe des Pixels in der Mitte des Bildes ist, die bei einer Dosis und bei Referenzbestrahlungsbedingungen erhalten wird, $D_{10}^{eq}$ eine vorbestimmte konstante Dosis ist und $\alpha, \beta$ Koeffizienten sind, die zuvor in einer vorherigen Kalibrierungsphase bestimmt wurden.

**2.** Verfahren zum Abschätzen einer Dosis nach Anspruch 1, **dadurch gekennzeichnet, dass** vor Schritt (b) von dem Portalbild das Hintergrundrauschen entfernt wird (620) durch Subtrahieren eines Bildes, das bei fehlender Bestrahlung aufgenommen wurde, um ein rauschbereinigtes Portalbild zu erhalten.

**3.** Verfahren zum Abschätzen einer Dosis nach Anspruch 2, **dadurch gekennzeichnet, dass** vor Schritt (b) das rauschbereinigte Bild korrigiert wird (630), indem es von einer Komponente befreit wird, die auf die Rückstreuung des Strahlenbündels durch die Umgebung des Portalbildgebers zurückzuführen ist.

**4.** Verfahren zum Abschätzen einer Dosis nach Anspruch 3, **dadurch gekennzeichnet, dass** die vorherige Kalibrierungsphase umfasst:

- einen Schritt (810) des Erfassens einer Mehrzahl $K$ von Portalbildern für eine Mehrzahl von Dosen $D^k$, $k = 1, ..., K,$ und bei Referenzbestrahlungsbedingungen;
- einen Schritt (820) des Beseitigens von Rauschen durch Subtrahieren eines Bildes von den Portalbildern, das bei fehlender Bestrahlung erfasst wird, um eine Mehrzahl $K$ von rauschbereinigten Bildern zu erhalten;
- einen Schritt (830) des Korrigierens der rauschbereinigten Bilder, um sie von einer Komponente zu befreien, die auf die Rückstreuung durch die Umgebung des Bildgebers zurückzuführen ist;

- einen Schritt (840) des Berechnens der Koeffizienten $\alpha$, $\beta$ ausgehend von den Werten $\frac{D^k}{D_{10}^{eq}}$ und ln $\left(\frac{N_g^k}{N_{g,10}}\right)$, $k = 1, ..., K.$

5. Verfahren zum Abschätzen einer Dosis nach Anspruch 4, **dadurch gekennzeichnet, dass** die Koeffizienten $\alpha$, $\beta$ ausgehend von den Werten $\frac{D^k}{D_{10}^{eq}}$ und $\ln\left(\frac{N_g^k}{N_{g,10}}\right)$, $k = 1, ..., K$ mittels einer linearen Regression erhalten werden.

6. Verfahren zum Abschätzen einer Dosis nach Anspruch 5, **dadurch gekennzeichnet, dass** die vorherige Kalibrierungsphase ferner eine Validierungsphase der Koeffizienten $\alpha$, $\beta$ einschießt, umfassend:

- einen Schritt (850) des Messens der zweidimensionalem Verteilung der abgegebenen Dosis in der Bezugstiefe für jede der Dosen, bei denen die Portalbilder erfasst wurden, wobei der Messschritt eine Mehrzahl $K$ von gemessenen Dosisbildern liefert;
- einen Schritt (860) des Umwandelns der erfassten Portalbilder in Dosisbilder, um eine Mehrzahl $K$ von errechneten Dosisbildern zu liefern,
- einen Schritt (870) des Vergleichens der gemessenen Dosisbilder mit den errechneten Dosisbildern, wobei die Koeffizienten $\alpha$, $\beta$ im Falle einer Übereinstimmung zwischen den einen und den anderen validiert werden.

7. Verfahren zum Abschätzen einer Dosis nach Anspruch 6, **dadurch gekennzeichnet, dass** jedes Portalbild $k = 1, ..., K$ in ein Dosisbild umgewandelt wird mittels der Beziehung

$$\frac{\hat{D}^k(i, j)}{D_{10}^{eq}} = \alpha + \beta \ln\left(\frac{N_g^k(i, j)}{N_{g,10}}\right)$$

worin $N_g^k(i, j)$ die Graustufe des Pixels $(i, j)$ des k-ten erfassten Portalbildes ist.

8. Verfahren zum Abschätzen einer Dosis nach Anspruch 6, **dadurch gekennzeichnet, dass** die gemessenen Dosisbilder und die errechneten Dosisbilder mittels einer Indexanalyse $\gamma$ verglichen werden.

9. Verfahren zum Überprüfen eines Behandlungsplans für ein externes Strahlentherapiesystem, das mit einem Portalbildgeber ausgestattet ist, wobei der Behandlungsplan durch eine Mehrzahl $M$ von zweidimensionalen Dosisverteilungen in einer Bezugstiefe definiert ist (700), wobei jede zweidimensionale Verteilung einem Einfallswinkel, einer Menge von Überwachungseinheiten und einer Strahlformung des Bestrahlungsbündels zugeordnet ist, wobei jede zweidimensionale Dosisverteilung ein Bild der Solldosis ergibt, wobei

- ein Portalbild jeweils für den Einfallswinkel, die Menge von Überwachungseinheiten und die Strahlformung des Bestrahlungsbündels erfasst wird, so dass eine gleiche Mehrzahl $M$ von erfassten Portalbildern erhalten wird, wobei das Verfahren **dadurch gekennzeichnet ist, dass**
- die erfassen Portalbilder in Dosisbilder umgewandelt werden, um eine Mehrzahl $M$ von errechneten Dosisbildern bereitzustellen, wobei die Umwandlung mittels des Terms

$$\frac{D^m(i, j)}{D_{10}^{eq}} = \alpha + \beta \ln\left(\frac{N_g^m(i, j)}{N_{g,10}}\right)$$

erfolgt, worin $D^m$ (i, j) die Graustufe des Pixels der Koordinaten (i, j) des Dosisbildes, m = 1, ..., M und $N_g^m (i, j)$ die Graustufe des Pixels der Koordinaten (i, j) des entsprechenden Portalbildes ist, $N_{g,10}$ die Graustufe des Pixels in der Mitte des Bildes ist, die bei einer Dosis und bei Referenzbestrahlungsbedingungen erhalten wird, $D_{10}^{eq}$ eine vorbestimmte konstante Dosis ist und $\alpha$, $\beta$ Koeffizienten sind, die zuvor in einer vorherigen Kalibrierungsphase bestimmt wurden;
- die errechneten Dosisbilder mit den Bildern der Solldosis verglichen werden.

10. Verfahren zum Überprüfen eines Behandlungsplans nach Anspruch 9, **dadurch gekennzeichnet, dass** vor dem Schritt der Umwandlung von den erfassten Portalbildern das Hintergrundrauschen entfernt wird (720) durch Subtrahieren eines Bildes, das bei fehlender Bestrahlung aufgenommen wurde.

11. Verfahren zum Überprüfen eines Behandlungsplans nach Anspruch 10, **dadurch gekennzeichnet, dass** vor dem Schritt der Umwandlung die so rauschbereinigten erfassten Portalbilder korrigiert werden (730), indem sie von einer Komponente befreit werden, die auf die Rückstreuung des Strahlenbündels durch die Umgebung des Portalbildgebers zurückzuführen ist.

12. Verfahren zum Überprüfen eines Behandlungsplans nach Anspruch 9, **dadurch gekennzeichnet, dass** das Vergleichen zwischen den berechneten Dosisbildern und den Solldosisbildern mittels einer Indexanalyse $\gamma$ erfolgt.

13. Verfahren zum Überprüfen eines Behandlungsplans nach einem der Ansprüche 9 bis 12, **dadurch gekennzeichnet, dass** die vorherige Kalibrierungsphase umfasst:

- einen Schritt (810) des Erfassens einer Mehrzahl $K$ von Portalbildern für eine Mehrzahl von Dosen $D^k$, k = 1, ..., $K$, und bei Referenzbestrahlungsbedingungen;
- einen Schritt (820) des Beseitigens von Rauschen durch Subtrahieren eines Bildes von den Portalbildern, das bei fehlender Bestrahlung erfasst wird, um eine Mehrzahl $K$ von rauschbereinigten Bildern zu erhalten;
- einen Schritt (830) des Korrigierens der rauschbereinigten Bilder, um sie von einer Komponente zu befreien, die auf die Rückstreuung durch die Umgebung des Bildgebers zurückzuführen ist;
- einen Schritt (840) des Berechnens der Koeffizienten $\alpha$, $\beta$ ausgehend von den Werten $\frac{D^k}{D_{10}^{eq}}$ und $\ln\left(\frac{N_g^k}{N_{g,10}}\right)$, k = 1, ..., $K$.

14. Verfahren zum Überprüfen eines Behandlungsplans nach Anspruch 13, **dadurch gekennzeichnet, dass** die Koeffizienten $\alpha$, $\beta$ ausgehend von den Werten $\frac{D^k}{D_{10}^{eq}}$ und $\ln\left(\frac{N_g^k}{N_{g,10}}\right)$, k = 1, ..., $K$ mittels einer linearen Regression berechnet werden.

**Claims**

1. Method for estimating the dose administered by an external radiotherapy system provided with a portal imaging device, **characterised in that**

(a) a portal image is acquired (610) using the portal imaging device;
(b) the grey level, $N_g$, of the pixel is determined (640) at the centre of the portal image, corresponding to the intersection of the portal imaging device with the axis of the irradiation beam;
(c) the dose administered by the system is calculated (650) at a depth of reference ($z_{ref}$) in water using

$$\frac{D}{D_{10}^{eq}} = \alpha + \beta \ln\left(\frac{N_g}{N_{g,10}}\right)$$

where $N_{g,10}$ is the grey level of the pixel at the centre of the image, obtained for a dose and reference irradiation conditions, $D_{10}^{eq}$ is a predetermined constant dose and $\alpha$, $\beta$ are coefficients

determined beforehand in a prior phase of calibration.

2. Method for estimating the dose according to claim 1, **characterised in that**, prior to the step (b) the background noise is removed (620) from the portal image by subtracting from it an image acquired in the absence of irradiation, in order to obtain a noise-suppressed portal image.

3. Method for estimating the dose according to claim 2, **characterised in that**, prior to the step (b), the noise-suppressed image is corrected (630) by removing from it a component due to the backscattering of the beam by the environment of the portal imaging device.

4. Method for estimating the dose according to claim 3, **characterised in that** said prior phase of calibration comprises:

   - a step (810) of acquiring a plurality $K$ of portal images for a plurality of doses, $D^k, k = 1,..., K,$ and in reference irradiation conditions;
   - a step (820) of removing the noise by subtracting from said portal images an image acquired in the absence of irradiation in order to obtain a plurality $K$ of noise-suppressed images;
   - a step (830) of correcting noise-suppressed images in order to remove from them a component due to the backscattering by the environment of the imaging device;
   - a step (840) of calculating coefficients $\alpha, \beta$ from values $\dfrac{D^k}{D_{10}^{eq}}$ and $\ln\left(\dfrac{N_g^k}{N_{g,10}}\right),$ $k = 1,..., K.$

5. Method for estimating the dose according to claim 4, **characterised in that** the coefficients $\alpha, \beta$ are obtained from values $\dfrac{D^k}{D_{10}^{eq}}$ and $\ln\left(\dfrac{N_g^k}{N_{g,10}}\right),$ $k = 1,..., K$ using linear regression.

6. Method for estimating the dose according to claim 5, **characterised in that** said prior phase of calibration can further include a phase of validation of coefficients $\alpha, \beta$ comprising:

   - a step (850) of measuring the two-dimensional distribution of a dose administered at the depth of reference for each one of the doses at which the portal images were acquired, said step of measuring supplying a plurality $K$ of measured dose images;
   - a step (860) of transforming acquired portal images into dose images, in order to supply a plurality $K$ of calculated dose images;
   - a step (870) of comparing said measured dose images with said calculated dose images, with the coefficients $\alpha, \beta$ being validated in the event of a concordance between the first and the second.

7. Method for estimating the dose according to claim 6, **characterised in that** each portal image $k = 1,..., K$ is transformed into a dose image using the relationship $\dfrac{\hat{D}^k(i,j)}{D_{10}^{eq}} = \alpha + \beta \ln\left(\dfrac{N_g^k(i,j)}{N_{g,10}}\right)$ where $N_g^k(i,j)$ is the grey level of the pixel $(i, j)$ of the $k$ th portal image acquired.

8. Method for estimating the dose according to claim 6, **characterised in that** the measured dose images and the calculated dose images are compared using an analysis of index $\gamma$.

9. Method for verifying a treatment plan for an external radiotherapy system provided with a portal imaging device, with the treatment plan being defined (700) by a plurality $M$ of two-dimensional distributions of a dose at a depth of reference, with each two-dimensional distribution being associated with an angle of incidence, a quantity of monitor units and a conformation of the irradiation beam, with each two-dimensional distribution of a dose giving a nominal dose image, said method being **characterised in that**:

   - a portal image is acquired for each angle of incidence, quantity of monitor units and conformation of the irradiation beam, in such a way as to obtain the same plurality $M$ of acquired portal images:
said method being **characterized in that**:

- the acquired portal images are transformed into dose images in order to supply a plurality $M$ of calculated dose images, said transform being obtained by the relationship $\dfrac{D^m(i, j)}{D_{10}^{eq}} = \alpha + \beta \ln\left(\dfrac{N_g^m(i, j)}{N_{g,10}}\right)$ where

$D^m(i, j)$ is the grey level of the pixel of coordinates $(i, j)$ of the dose image, $m = 1,..., M$, and $N_g^m(i, j)$ is the grey level of the pixel of coordinates $(i, j)$ of the corresponding portal image, $N_{g,10}$ is the grey level of the pixel at the centre of the image, obtained for a dose and reference irradiation conditions, $D_{10}^{eq}$ is a predetermined constant dose and $\alpha, \beta$ are coefficients determined beforehand in a prior phase of calibration;
- said calculated dose images are compared to the nominal dose images.

10. Method for verifying a treatment plan according to claim 9, **characterised in that**, prior to the step of transforming, the background noise is removed (720) from the acquired portal images by subtracting from them an image acquired in the absence of irradiation.

11. Method for verifying a treatment plan according to claim 10, **characterised in that**, prior to the step of transforming, the acquired portal images noise-suppressed as such are corrected (730), by removing from them a component due to the backscattering of the beam by the environment of the portal imaging device.

12. Method for verifying a treatment plan according to claim 9, **characterised in that** the comparison between the calculated dose images and the nominal dose images is carried out using an analysis of index $\gamma$.

13. Method for verifying a treatment plan according to one of claims 9 to 12, **characterised in that** prior phase of calibration comprises:

- a step (810) of acquiring a plurality $K$ of portal images for a plurality of doses, $D^k$, $k = 1,..., K$, and in reference irradiation conditions;
- a step (820) of removing noise by subtracting from said portal images an image acquired in the absence of irradiation in order to obtain a plurality $K$ of noise-suppressed images;
- a step (830) of correcting noise-suppressed images in order to remove from them a component due to the backscattering by the environment of the imaging device;

- a step (840) of calculating coefficients $\alpha, \beta$ from values $\dfrac{D^k}{D_{10}^{eq}}$ and $\ln\left(\dfrac{N_g^k}{N_{g,10}}\right)$, $k = 1,..., K$.

14. Method for verifying a treatment plan according to claim 13, **characterised in that** the coefficients $\alpha, \beta$ are calculated

from values $\dfrac{D^k}{D_{10}^{eq}}$ and $\ln\left(\dfrac{N_g^k}{N_{g,10}}\right)$, $k = 1,..., K$ using linear regression.

**FIG.1**

photon (X)

200

hv

Cu

210

e⁻

photons (visible)

$Gd_2O_2S : Tb$

220

a − Si

230

## FIG.2

S

Ω

C × C

FIG.3A

S

100 cm

$z_{eq}$

C × C

FIG.3B

FIG.4A  FIG.4B  FIG.4C  FIG.4D

FIG.5A  FIG.5B  FIG.5C

acquisition de l'image portale
$N_g(i, j)$ — 610

élimination du bruit de fond — 620

élimination de la composante rétrodiffusée — 630

détermination du niveau de gris du pixel central, $N_g$ — 640

calcul de la dose délivrée
$$\frac{D}{D_{10}^{eq}} = \alpha + \beta \ln\left(\frac{N_g}{N_{g,10}}\right)$$ — 650

# FIG.6

acquisition des images portales
$N_g^m(i, j)$; $i, j = 1,... N$ ; $m = 1,...,M$ ⎯710

élimination du bruit de fond ⎯720

élimination de la composante rétrodiffusée ⎯730

calcul des images en dose à partir des images acquises

$$\frac{D^m(i, j)}{D_{10}^{eq}} = \alpha + \beta \ln \left( \frac{N_g^m(i, j)}{N_{g,10}} \right)$$

⎯740

planification de traitement
obtention des images en dose de consigne
$D^m(i, j)$, $i, j = 1,... N$ ; $m = 1,...,M$ ⎯700

comparaison des images en dose calculées avec les images en dose de consigne ⎯750

validation du plan de traitement si correspondance ⎯760

FIG.7

acquisition d'une pluralité *K* d'images portales pour une pluralité de doses   810

élimination du bruit de fond   820

élimination de la composante rétrodiffusée   830

calcul par régression linéaire des coefficients $\alpha, \beta$ sur les valeurs

$$\frac{D^k}{D_{10}^{eq}} \quad \text{et} \quad \ln\left(\frac{N_g^k}{N_{g,10}}\right)$$

840

mesure des distributions bidimensionnelles de dose   850

transformation des images portales en images en dose calculées

$$\frac{\hat{D}^k(i,j)}{D_{10}^{eq}} = \alpha + \beta \ln\left(\frac{N_g^k(i,j)}{N_{g,10}}\right)$$

860

comparaison des images en dose mesurées et des images en dose calculées validation des coefficients si concordance   870

## FIG.8

FIG.9

EP 3 145 587 B1

FIG.10

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2248551 A

- US 8130905 B

**Littérature non-brevet citée dans la description**

- **VAN ESCH et al.** The use of an a Si-based EPID for routine absolute dosimetry pre-treatement verification of dynamic IMRT fields. *Radiotherapy and Oncology,* 2004, vol. 71, 222-234
- **MCDERMOTT et al.** Dose response and ghosting effects of an amorphous silicon electronic portal imaging device. *Med. Phys.,* Février 2004, vol. 31 (2
- **P. WINKLER et al.** Data response characteristics of an amorphous silicon EPID. *Med. Phys.,* Octobre 2005, vol. 32 (10), 3095-3105

- **P. WINKLER et al.** Implementation and validation of portal dosimetry with an amorphous silicon EPID in the energy range from 6 to 25 MV. *Phys. Med. Biol.,* 2007, vol. 52, N355-N365
- **B.W. KING et al.** A method for removing arm backscatter from EPID images. *Med. Phys.,* Juillet 2013, vol. 40 (7), 071703-1, 071703-9
- **D.A. LOW et al.** A technique for the quantitative evaluation of dose distributions. *Med. Phys.,* vol. 25 (5), 656-661